Europäisches Patentamt

European Patent Office

Office européen des brevets

⑱

(11) Publication number: **0 156 110
B1**

(12) # EUROPEAN PATENT SPECIFICATION

(45) Date of publication of patent specification: **18.10.89**

(21) Application number: **85100677.5**

(22) Date of filing: **24.01.85**

(51) Int. Cl.⁴: **A 61 M 1/36,** F 16 L 55/14, F 16 K 7/02

(54) Valve means.

(30) Priority: **09.02.84 SE 8400682**

(43) Date of publication of application:
**02.10.85 Bulletin 85/40**

(45) Publication of the grant of the patent:
**18.10.89 Bulletin 89/42**

(84) Designated Contracting States:
**BE CH DE FR GB IT LI LU NL**

(56) References cited:
**EP-A-0 111 696
DE-B-2 209 322
US-A-3 697 418
US-A-4 425 113
US-A-4 428 745**

(73) Proprietor: **Gambro Lundia AB
Box 10101
S-220 10 Lund (SE)**

(72) Inventor: **Claren, Jan Seivert
Protokollgränden 38
S-222 47 Lund (SE)**
Inventor: **Johansson, Bo Ingemar, Willy
Lokföraregatan 3 D
S-222 37 Lund (SE)**

(74) Representative: **Boberg, Nils Gunnar Erik
Gambro AB Patent Department Box 10101
S-220 10 Lund (SE)**

Courier Press, Leamington Spa, England.

## Description

This invention relates to a valve means which comprises at least three connections intended to serve as inlets and/or outlets, the connections being provided in communication with a channel system which is formed of flexible walls and which is compressible from outside to provide an optional communication between selected connections. Such a valve means is known from US—A—4428745.

Such valve means are useful for directing liquid flows in flow circuits of for example the kind which is described in U.S. Patent 3 697 418 and in EP—A2—0 111 696.

Examples of known valve means of the above described kind are illustrated in said two references and in DE—B—22 09 322, US—A—4 425 113 and US—A—4 428 745. Common to these known valve means is that they comprise one or a number of movable parts in a channel system, which is/are movable between two fixed positions to provide communication between the respective channels and outer connections which form parts of a flow circuit.

Due to the fact that these known valve means use movable parts to open and close, respectively, flow communications it is obvious that the risk of leakage due to wear of for example the movable part often increases as the number of uses increases.

A further disadvantage is that the movable part (which also may fulfil a sealing function) often must be manufactured with very small dimensional tolerances, whereby the manufacture of said known valve means necessarily is increased.

The above three last mentioned references include the improvement that no movable parts are situated in the channel system. Instead said channel system include flexible walls which are compressible from the outside. Said walls are, however, provided by conventional tubings which have to be connected by different rather complicated connecting means.

An object of the present invention is therefor to provide an improved valve means including a very simple channel system.

This object is achieved in that said channel system is formed from and between two flexible flat sheets which are welded or glued together.

The channels of said channel system between said flat sheets may be provided through welding or gluiding along any suitable pattern which is determined inter alia by the number of used connections.

For example if the number of connections is four, such as two inlets and two outlets, the channel system has preferably the shape of one single closed annular channel. The respective inlets and outlets are thereby preferably located diametrically opposite to each other, as explained in the following.

The proper connections are preferably constituted by tube nipples being welded to glued between said flat sheets and being manufactured from a plastic material which is weldable and gluable, respectively, with said flat sheets.

In this embodiment with four connections said channel system may also be constituted by the total space which is formed between said two flat sheets which are welded or glued together along their circumferential edge lines.

The valve means according to the present invention may be used in any liquid flow system in which the respective liquid flows are to be directed in an optional variable manner. It is however particularly suitable for use in a so-called extracorporeal system for treatment of for example blood to alternatingly direct the blood and a eluting agent (rinsing solution), respectively, to either of two used adsorption columns in the system.

The invention will be described more in detail in the following with reference to the accompanying drawings in which

Fig. 1 is a schematic view of the valve means according to a preferred embodiment,

Fig. 2 is a view taken long line II—II in Fig. 1, and

Fig. 3 illustrates the use of said valve means in an outer flow system.

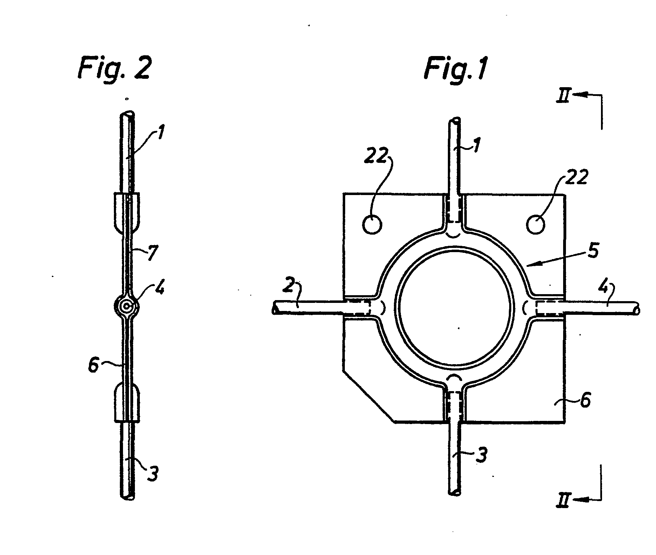

As is shown in Fig. 1 a preferred valve means according to the invention thus comprises four individual connections 1—4 in communication with an annular substantially closed channel 5 formed between two welded flat sheet 6 and 7 (Fig. 2). In the illustrated example two of said connections are intended to serve as inlets, such as 1 and 3, and two as outlets, such as 2 and 4.

The inlets 1 and 3 and the outlets 2 and 4 are conveniently provided in pairs diametrically opposed to each other and may be constituted by short tube nipples welded between the flat sheets 6 and 7.

The channel 5 needs not be endless, as is shown in Fig. 1 but can as well be in the form of an interrupted loop, such as a horseshoe with preferably two of said connections being provided at each end of said interrupted loop.

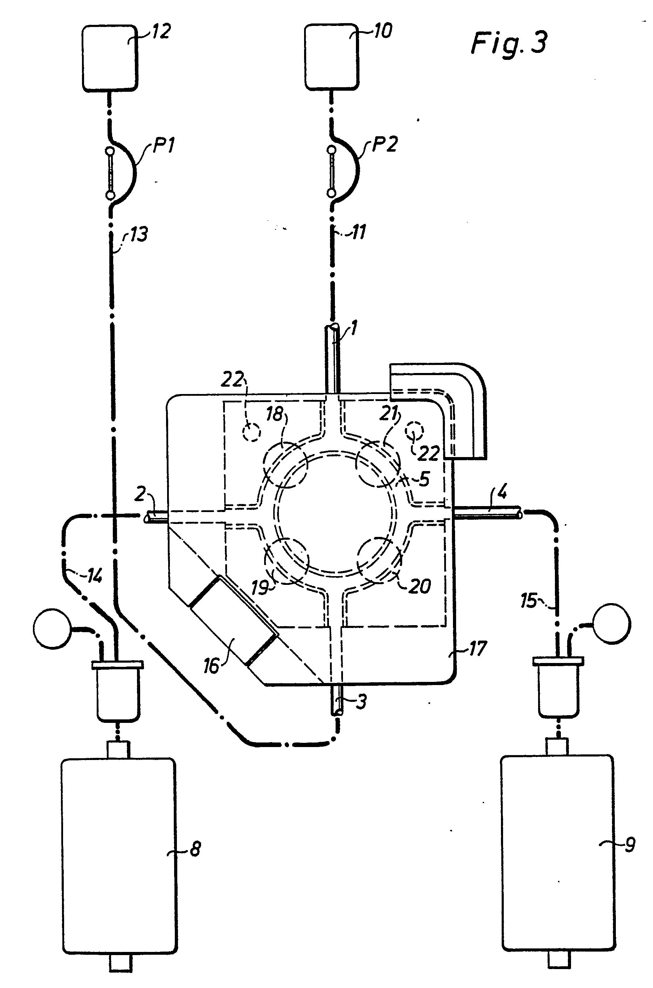

The flow system according to Fig. 3 may for example be a so-called extracorporeal system for treatment of blood, which is intended for example to remove specific antibodies from the blood with use of two parallelly coupled adsorption columns 8 and 9 through which the blood is intended to flow for adsorption of such antibodies.

Conveniently the columns therefore comprise antigens which are immobilized on any suitable carrier and which are specifically directed against said antibodies. Such columns are previously known and need therefore not be described in detail herein. Instead it is referred to for example the Swedish Patent Application No. 8206520-2, filed November 16, 1982, which describes such an adsorption column particularly intended to remove antibodies against factor IX from blood of a hemophilic patient.

In the illustrated treating system (intended to remove for example such antibodies against factor IX from blood of a hemophilic patient) the first inlet 1 is in communication with a source 10 for an eluting agent (rinsing liquid) via an outer connection 11, while the other inlet 3 in a similar way is in communication with an outer source 12 for blood, for example a patient, via an outer connection 13. Said first outlet 2 is in communication with a first adsorption column 8 via an outer connection 14 and the other outlet 4 is in communication with a second column 9 via an outer connection 15.

Preferably the valve means is located behind a door 17 which is openable and closable by means of for example a hinge 16 and which is mounted for example on the front panel of a monitoring unit which is forming a part of the treating system. The channel 5 can thereby be externally compressed by means of compressing organs 18—21 (as schematically illustrated by broken circles in Fig. 3), which can be adapted to either alone or in pairs be directed to externally compress the channel 5 to control liquid flows in a desired manner, as will be explained in the following in connection with the description of the operation of the valve means.

If, for example, the channel communications between the first inlet 1 and the first outlet 2 and between the second inlet 3 and the second outlet 4, respectively, are kept closed by compression by means of the organs 18 and 19, respectively, and if in a corresponding way the channel communications between the second inlet 3 and the first outlet 2 and the first inlet 1 and the second outlet 4, respectively, are kept open, the blood is thus pumped from the source 12 by means of a pump P1 in the outer connection 13 into a channel 5 through the second outlet 3. From there the blood is pumped to the outlet 2 and reaches the first absorption column 8 via the outer connection 14 for adsorption of said antibodies. In a corresponding manner eluting liquid is thus pumped from the source 10 by means of a pump P2 in the outer connection 11 into the channel 5 through the first inlet 1 and further to the second column 9, which previously can be saturated on said antibodies, via the second outlet 4 and the outer connection 15 for regenerating of the second column 9.

When the first column 8 is saturated on antibodies the compressing organs 18 and 20 open the respective channel communications at the same time as the compressing organs 19 and 21 from outside clamp the channel communications between the second inlet 3 and the first outlet 2 and the first inlet 1 and the second outlet 4, respectively. In this position the blood is instead pumped via the second inlet 3 to the second column 9 via the second outlet 4 and the outer connection 15, while the eluting agent is pumped to the first column 8 via the first inlet 1 and the first outlet 2 and the outer connection 14. When the second column 9 is again saturated on antibodies the flow directions are again changed for the blood and the eluting agent by means of the compressing organs 18—21 as described above, whereby the alternating adsorption and regeneration is repeated.

As previously mentioned said compressing organs 18—21 need not be directed in pairs, but this can be directed independently from each other, whereby it is possible to direct the liquid flows optionally in an unlimited number of optional ways.

According to the invention said flat sheets 6 and 7 may form part of a disposable tube set further comprising pumping segments for extracorporeal treatment of blood.

As suggested in Figs. 1 and 3 (at 22) said flat sheets may comprise means for suspension against the door 17, whereby the flat sheets preferably have an asymmetric outer shape intended to facilitate location of the flat sheets to thereby avoid a wrong use of these.

## Claims

1. Valve means comprising at least three connections (1—4) intended to serve as inlets (1 and 3) and/or outlets (2 and 4), the connections (1—4) being provided in communication with a channel system (5) which is formed of flexible walls (6 and 7) and which is compressible from outside to provide an optional communication between selected connections, characterized in that said channel system (5) is formed from and between two flexible flat sheets (6 and 7) which are welded or glued together.

2. Valve means according to claim 1 characterized in that said connections (1—4) are provided in communication with outer connections (11, 14, 13, 15) of a flow circuit.

3. Valve means according to any of the preceding claims, characterized in that said channel system (5) being in the form of an endless annular channel or of an interrupted loop.

4. Valve means according to claim 3, characterized in that said inlets (1 and 3) and said outlets (2 and 4) are provided diametrically opposed to each other.

5. Valve means according to any of the preceding claims, characterized in that said connections (1—4) are constituted by tube nipples which are welded or glued between said flat sheets (6 and 7).

6. Valve means according to any of the preceding claims, characterized in that said flat sheets (6 and 7) comprise means (22) for suspension against a support (17) against which said channel system (5) is intended to be compressed.

7. Valve means according to claim 6, characterized in that said flat sheets (6 and 7) have an asymmetric outer shape for locating against said support (17).

8. Valve means according to any of the preceding claims, characterized by comprising compressing organs (18—21), for example in the form of movable pistons for compression of said channel system (5).

## Patentansprüche

1. Ventileinrichtung mit zumindest drei Anschlüssen (1—4), welche als Einlässe (1 und 3) und/oder Auslässe (2 und 4) dienen sollen, wobei die Anschlüsse (1—4) in Verbindung mit einem Kanalsystem (5) stehen, welches aus flexiblen Wänden (6 und 7) gebildet ist und welches von außen her zusammendrückbar ist, um eine gewünschte Verbindung zwischen ausgewählten Anschlüssen herzustellen, dadurch gekennzeichnet, daß das Kanalsystem aus zwei flexiblen, flachen Lagen (6 und 7) und zwischen diesen gebildet ist, welche zusammengeschweißt oder geklebt sind.

2. Ventileinrichtung nach Anspruch 1, dadurch gekennzeichnet, daß die Anschlüsse (1—4) in Verbindung mit äußeren Anschlüssen (11, 14, 13, 15) eines Strömungskreises vorgesehen sind.

3. Ventileinrichtung nach einem der vorstehenden Ansprüche, dadurch gekennzeichnet, daß das Kanalsystem (5) die Form eines endlosen ringförmigen Kanals oder einer unterbrochenen Schleife hat.

4. Ventilmittel nach Anspruch 3, dadurch gekennzeichnet, daß die Einlässe (1 und 3) und die Auslässe (2 und 4) einander diametral gegenüberliegend angeordnet sind.

5. Ventileinrichtung nach einem der vorstehenden Ansprüche, dadurch gekennzeichnet, daß die Anschlüsse (1—4) von Schlauchnippeln gebildet werden, welche zwischen den flachen Bahnen (6 und 7) eingeschweißt oder eingeklebt sind.

6. Ventileinrichtung nach einem der vorstehenden Ansprüche, dadurch gekennzeichnet, daß die flachen Lagen (6 und 7) Mittel (22) zum Aufhängen an einer Halterung (176 aufweisen, gegen welche das Kanalsystem (5) zusammengepreßt werden soll.

7. Ventileinrichtung nach Anspruch 6, dadurch gekennzeichnet, daß die flachen Lagen (6 und 7) eine asymmetrische äußere Form haben, um sie an der Halterung (17) anzuordnen.

8. Ventileinrichtung nach einem der vorstehenden Ansprüche, dadurch gekennzeichnet, daß sie Druckorgane (18—21) aufweist, beispielsweise in Form beweglicher Kolben zum Zusammendrükken des Kanalsystems (5).

**Revendications**

1. Dispositif de soupape comprenant au moins trois connexions (1—4) destinées à servir d'entrées (1 et 3) et/ou de sorties (2 et 4), les connexions (1—4) étant prévues en communication avec un système de canaux (5) qui est défini par des parois flexibles (6 et 7) et qui est compressible de l'extérieure pour engendrer une communication optionnelle entre des connexions sélectionnées, caractérisé en ce que ledit système de canaux (5) est formé par et entre deux feuilles planes flexibles (6 et 7), qui sont soudées ou collées l'une à l'autre.

2. Dispositif de soupape suivant la revendication 1, caractérisé en ce que lesdites connexions (1—4) sont prévues en communication avec des liaisons extérieures (11, 14, 13, 15) d'un circuit de fluides.

3. Dispositif de soupape suivant l'une quelconque des revendications précédentes, caractérisé en ce que ledit système de canaux (5) est sous la forme d'un canal annulaire sans fin ou d'une boucle interrompue.

4. Dispositif de soupape suivant la revendication 3, caractérisé en ce que lesdites entrées (1 et 3) et lesdites sorties (2 et 4) sont disposées en opposition diamétrale mutuelle.

5. Dispositif de soupape suivant l'une quelconque des revendications précédentes, caractérisé en ce que lesdites connexions (1—4) sont constituées par des raccords tubulaires qui sont soudées ou collées entre lesdites feuilles planes (6 et 7).

6. Dispositif de soupape suivant l'une quelconque des revendications précédentes, caractérisé en ce que lesdites feuilles planes (6 et 7) comprennent des moyens (22) pour la suspension à un support (17) contre lequel ledit système de canaux (5) doit être comprimé.

7. Dispositif de soupape suivant la revendication 6, caractérisé en ce que lesdites feuilles planes (6 et 7) ont une forme extérieure asymétrique, pour la mise en place correcte contre ledit support (17).

8. Dispositif de soupape suivant l'une quelconque des revendications précédentes, caractérisé en ce qu'il comprend des organes de compression (18—21), par exemple sous la forme de pistons mobiles pour la compression dudit système de canaux (5).

# Fig. 2

# Fig.1

*Fig.3*